# EUROPEAN PATENT SPECIFICATION

(11) **EP 4 494 101 B1**
(45) Date of publication and mention of the grant of the patent: **02.09.2026**
(21) Application number: 23707411.7
(22) Date of filing: 01.03.2023
(51) Int. Cl.: G06T 7/33

(54) **METHOD FOR MODELLING A JOINT**
VERFAHREN ZUR MODELLIERUNG EINES GELENKS
PROCÉDÉ DE MODÉLISATION D'UNE ARTICULATION

(30) Priority: 14.03.2022 EP 22161849
(43) Date of publication of application: 22.01.2025
(73) Proprietor: Koninklijke Philips N.V., 5656 AE Eindhoven (NL)
(72) Inventor: GOOSSEN, André, 5656 AG Eindhoven (NL); BYSTROV, Daniel, 5656 AG Eindhoven (NL); KROENKE-HILLE, Sven, 5656 AG Eindhoven (NL); BRUECK, Heiner, Matthias, 5656 AG Eindhoven (NL); VON BERG, Jens, 5656AG Eindhoven (NL); YOUNG, Stewart, Matthew, 5656 AG Eindhoven (NL)
(74) Representative: Philips Intellectual Property & Standards
(86) International application number: PCT/EP2023/055098
(87) International publication number: WO 2023/174681

(56) References cited:
- WO-A1-2015/089118
- CHEN XIN ET AL: "Automatic Generation of Statistical Pose and Shape Models for Articulated Joints", IEEE TRANSACTIONS ON MEDICAL IMAGING, IEEE, USA, vol. 33, no. 2, 1 February 2014 (2014-02-01), pages 372 - 383, XP011538599, ISSN: 0278-0062, [retrieved on 20140130], DOI: 10.1109/TMI.2013.2285503
- MASCHINO E ET AL: "Joint registration and averaging of multiple 3D anatomical surface models", COMPUTER VISION AND IMAGE UNDERSTANDING, ACADEMIC PRESS, US, vol. 101, no. 1, 1 January 2006 (2006-01-01), pages 16 - 30, XP024917626, ISSN: 1077-3142, [retrieved on 20060101], DOI: 10.1016/J.CVIU.2005.06.004
- CONSTANTINESCU MIHAELA A M ET AL: "Constrained Statistical Modelling of Knee Flexion From Multi-Pose Magnetic Resonance Imaging", IEEE TRANSACTIONS ON MEDICAL IMAGING, IEEE, USA, vol. 35, no. 7, 1 July 2016 (2016-07-01), pages 1686 - 1695, XP011615416, ISSN: 0278-0062, [retrieved on 20160629], DOI: 10.1109/TMI.2016.2524587
- FLEUTE M ET AL: "NONRIGID 3-D/2-D REGISTRATION OF IMAGES USING STATISTICAL MODELS", ELECTRONIC PUBLISHING, ARTISTIC IMAGING, AND DIGITAL TYPOGRAPHY; [LECTURE NOTES IN COMPUTER SCIENCE , ISSN 0302-9743], SPRINGER VERLAG, DE, 19 September 1999 (1999-09-19), pages 138 - 147, XP008010626, ISBN: 978-3-540-24128-7, DOI: 10.1007/10704282_15
- HANSIS EBERHARD ET AL: "Landmark constellation models for medical image content identification and localization", INTERNATIONAL JOURNAL OF COMPUTER ASSISTED RADIOLOGY AND SURGERY, SPRINGER, DE, vol. 11, no. 7, 11 December 2015 (2015-12-11), pages 1285 - 1295, XP035990794, ISSN: 1861-6410, [retrieved on 20151211], DOI: 10.1007/S11548-015-1328-5

## Description

### FIELD OF THE INVENTION

The invention provides a computer-implemented method for modelling a joint, a data processing system, a system comprising the data processing system, a computer program product, and a computer readable medium.

### BACKGROUND OF THE INVENTION

Many recent developments in medical image analysis and processing demand dynamic anatomical models, also referred to as articulated models, e.g., representative of postures such as flexion of a joint or inhale/exhale for the thorax.

For example, 3D joint models may be employed for quality assessment, preparing for diagnosis, and/or treatment planning. In particular, certain diagnostic questions require a specific image acquisition whereby the joint flexion is within a specific range, since deviation from this flexion might cause certain anatomical features of diagnostic interest to be obscured within projection images.

Articulated models may be used to evaluate the positioning of the subject based upon just the image data and, once they are fitted to available data, also allow for judging joint dynamics and (ab)normality of movement range.

Building those articulated models over a wide patient population is tedious and time-consuming.

Moreover, building them requires the use of imaging modalities, e.g., using ionizing radiation, that may be harmful to a patient. Accordingly, volunteer scans outside of regular imaging sessions, like routine exams of a patient, may not be available at all in sufficient number and also pose ethical concerns.

Regular imaging sessions, like routine exams of a patient do usually not allow for significant workflow changes, particularly if they are time consuming, e.g., imaging a joint in multiple poses using the imaging modality used for the imaging session. Clinical workflows typically do not permit the addition of steps that slow down routine scheduling and throughput, such as performing dedicated measurements for different poses during examination. For example, externally measuring the flexion for multiple images obtained by the imaging modality of the workflow at different poses and recording it to be stored with the image, so as to co-register and sort them according to flexion angle and, based thereon, build a model is difficult to incorporated into the routine workflows.

Accordingly, it may be difficult to build a complete model with adequate precision.

The scientific publications by CHEN XIN ET AL: "Automatic Generation of Statistical Pose and Shape Models for Articulated Joints", IEEE TRANSACTIONS ON MEDICAL IMAGING, vol. 33, no. 2, 1 February 2014, pages 372-383, ISSN: 0278-0062, DOI: 10.1109/TM1.2013.2285503 and CONSTANTINESCU MIHAELA A M ET AL: "Constrained Statistical Modelling of Knee Flexion From Multi-Pose Magnetic Resonance Imaging", IEEE TRANSACTIONS ON MEDICAL IMAGING, vol. 35, no. 7, 1 July 2016, pages 1686-1695, ISSN: 0278-0062, DOI: 10.1109/TMI.2016.2524587, represent background art relating to articulated joint modelling in medical image data.

### SUMMARY OF THE INVENTION

It is an object of the present invention to allow for improved modelling of a joint.

The invention provides a method for modelling a joint, a data processing system, a system comprising the data processing system, a computer program product, and a computer readable medium according to the independent claims.

The method for modelling a joint according to the present disclosure comprises processing first 3D surface image data of a body part in a first pose by means of an automatic pose retrieval method so as to obtain first articulation parameters representative of the first pose, wherein the body part comprises a first anatomical structure and a second anatomical structure connected by a joint.

The method for modelling a joint according to the present disclosure further comprises performing image registration between first medical image data of the body part, the first medical image data acquired simultaneously with the first 3D surface image data and depicting the first anatomical structure and the second anatomical structure, and second medical image data of the body part in a second pose. The image registration is performed individually for each of the first anatomical structure and the second anatomical structure.

The method for modelling a joint according to the present disclosure further comprises determining transformation data representing one or more first transformations required to register the first anatomical structure in the first medical image data to the first anatomical structure in the second medical image data and/or one or more second transformations required to register the second anatomical structure in the first medical image data to the second anatomical structure in the second medical image data.

The method for modelling a joint according to the present disclosure further comprises creating and/or updating a statistical articulated joint model based at least on the transformation data, the first articulation parameters, and second articulation parameters representative of the second pose.

In other words, the limitations of known methods can be addressed by relying on additional sensor data (in addition to data from the imaging modality) providing depth information, for example from a surface scanner or range camera or other 3D camera system, e.g., a RGBD camera, to record pose information during examination with the imaging modality. This enables to relate pose and image from the imaging modality obtained in a routine workflow. Based on the relation, an articulated model can be built and/or within a standard workflow of a radiology department.

Particularly the method of the present disclosure allows to acquire arbitrary poses without the need for additional recording information. For example, no user observations regarding the articulation pose of a joint for a given acquisition are required and pose data does not have to be measured/tracked manually by the radiographer. This is particularly helpful when using measurement devices for measuring pose parameters would be a burden for the imaging modality, e.g. within a CT bore due to space restrictions or in an MRI scanner due to the use of metal not being possible.

Thus, since the proposed approach does not require interruption of imaging workflow, it allows for including images from clinical routine workflows to the data used for building the articulated model. Thus, a large and diverse dataset can go into building the model.

The present disclosure allows, as one example, for the development of articulated joint models, where the model is created using MR scans of a joint in which the joint has been positioned a priori in one or more known poses or articulations.

A potential application of the articulated model is for skeletal X-ray image quality assessment.

As can be seen from the above, the claim entails creating and/or updating the articulated model. For example, the method may entail using any articulated model, which may be empirical, semi-empirical, or theoretical, as a starting point and apply the steps of the present disclosure, particularly repeatedly for one or more subjects and/or one or more imaging sessions of the same subject or different subjects, to improve the model. Improving the model may concern different aspects. For example, the model may become less generic and more closely represent reality, particularly statistically over a wide range of subjects. Moreover, the precision of the model may be improved, e.g., by allowing to meaningfully increase the size of the data set. Moreover, the range of the model may be extended with respect to extreme poses towards the end of the movement spectrum, e.g., poses representative of a very strong flexion and/or poses representative of a hyperextension of a joint.

In the present disclosure, excluding the description of the prior art, the term "joint model" is used synonymously with the term "statistical articulated joint model" for the sake of readability.

A joint is a connection movably connecting bones in the body.

The body part may, for example, be an arm, a wrist, a shoulder, an ankle, a leg, a hip, the foot, the hand, or any other portions of the body comprising a joint.

In the present disclosure, the term "anatomical structure" refers to a sub-surface part of the body. An anatomical structure may, for example, be a bone, a muscle, a cartilage, a ligament, or the like. It is noted that different anatomical structures may be rigid to different degrees, such the movement of some anatomical structures may have more degrees of freedom than that of other anatomical structures. For example, a bone is essentially rigid and non-compressible, such that its movement can generally be represented by translations and rotations. Sinews or cartilage may be elastically deformed, e.g., lengthened, contracted, or compressed, so their movement may also include, particularly elastic, deformation and scaling.

A pose is a certain relative arrangement of a set of anatomical structures. For example, a pose may be defined by a relative arrangement of two bones, e.g., one or more angles describing their relative arrangement in one or more degrees of freedom.

The pose can be represented by articulation parameters. Articulation parameters may be any parameterization of relative arrangement of the anatomical structures. For example, articulation parameters may comprise flexion angles.

It is to be understood that the first and second pose are different, e.g., they differ in at least one articulation parameter. For example, the first pose and the second pose may differ in that they have a different flexion angle.

Automatic pose retrieval may be any method known in the art, e.g., from gaming applications, that determines the pose of body parts based on 3D surface image data. The 3D information allows for resolving directional ambiguities that might not be resolved otherwise.

It is noted that according to claim 1, second articulation parameters representative of the second pose are derived from second 3D surface image data of the body part obtained using automatic pose retrieval. In other examples, , second articulation parameters may be articulation parameters derived from the second medical image data and/or image data obtained using a different imaging modality and/or obtained by direct pose measurements.

3D surface image data is data that provides depth information. For example, 3D surface image data may be obtained, for example, by a 3D surface scanner, a stereoscopic camera, and/or an RGBD camera.

Medical image data, in the present disclosure, refers to image data depicting sub-surface structures of the body, e.g., image data obtained using ionizing radiation. The medical image data may, for example, comprise CT image data, MRI data, or X-ray image data. Medical image data may comprise 2D and/or 3D image data. 3D image data provides depth information of the imaged structure. 2D image data may, for example, depict a projection of the imaged structure.

According to the present disclosure, the first 3D surface image data and the first medical image data are acquired simultaneously. This may include that, during an imaging session, first 3D surface image data is acquired continuously over a period of time including the time or times when the first medical image data is acquired. 3D surface image data, particularly from said period of time may also be employed for building the model if there is not corresponding medical image data, e.g., when it was obtained in the time between capturing the medical images. For example, such image data may be used for improving automatic pose retrieval accuracy or for improving accuracy when building the joint model, e.g., by inferring keypoints of the anatomical structure from the surface image data, as explained in detail further below, or by aiding in removing ambiguities.

Image registration may be performed using registration methods known in the art, including 2D/3D registration, wherein 2D image data is registered with 3D image data and/or a mesh, and 3D/3D registration, wherein 3D image data is registered with 3D image data and/or a mesh.

According to the present disclosure, image registration is performed individually for each anatomical structure. This means that the first and the second anatomical structure are independently registered. The image registration may nonetheless be performed in parallel for the first and second anatomical structure.

Transformations according to the present disclosure may include rotations and/or translations. In addition, transformations may include deformations and scaling, e.g., in case the anatomical structure is non-rigid and/or compressible.

An articulated joint model may be a model that is representative of the movement of the joint, particularly the anatomical structures of the joint, between different articulations.

The articulated joint model may be a 2D model, which models the joint in a projection, or a 3D model having depth information of the joint, particularly of the anatomical structures. For the sake of completeness, even if the articulated joint model is a 2D model, the process of creating and/or updating the model involves 3D data of some kind so as to allow avoid ambiguities.

A statistical model is representative of data obtained from multiple imaging sessions, particularly, of more than one subject. As an example, the same joint can be imaged for multiple subjects and the model can be built so as to average out differences between different subjects, e.g., size and shape differences, differences in alignment of the anatomical structures or the like. The statistical model may also be selectively based on data from a plurality of subjects sharing certain characteristics, as will be described in detail below.

Creating a statistical articulated joint model may entail starting with no model of the overall joint, for example starting with individual models of the first and second anatomical structures, and/or starting with a rigid model of the joint and/or a non-statistical model of the joint (articulated or non-articulated) to obtain a statistical articulated joint model.

Updating a statistical articulated joint model may include modifying a current version of a statistical articulated joint model to account for additional information, in particular, to account for the transformation data, the first articulation parameters, and second articulation parameters representative of the second pose.

Accordingly, creating and/or updating the statistical articulated joint model based on transformation data, the first articulation parameters, and second articulation parameters representative of the second pose, may be seen as taking said data and parameters into account for modelling the joint, optionally together with pre-existing data for modelling the joint.

According to the present disclosure, the statistical articulated joint model may be based on transformation data, first articulation parameters, and second articulation parameters from image data acquired in multiple imaging sessions of a subject and/or from one or more imaging sessions of each of a plurality of subjects.

An imaging session may be a routine examination and may, in particular, not comprise changing the routine workflow, particularly may not comprise acquiring medical image data in addition to the ones that are part of the routine examination and may not comprise performing manual measurement of pose. By allowing to combine data from different imaging sessions of the same or different subjects, a large data set and, accordingly, accurate statistics can be performed, thereby allowing for a more accurate model.

Alternatively or in addition, according to the present disclosure, creating and/or updating the statistical articulated joint model may comprise determining a mean over a randomly selected subset of the population and/or determining a mean over a subset of the population sharing a type of misalignment of the joint.

When using data acquired from a plurality of, particularly randomly selected, subjects, a statistical model that averages out subject-specific characteristics can be provided.

When only data acquired for the subset of population sharing a type of misaligned is used for the statistical model, this may allow determining characteristics of the joint associated with the misalignment. This may help better address the needs brought about by the misalignment and yet still have large data set compared to only a single subject.

According to the present disclosure, the statistical articulated joint model may be a 3D model. For example, a 3D surface of each of the anatomical structures of the joint may be provided, particularly over the entire range of the model. This allows for a particularly detailed study of the anatomical structures and their interactions.

The method of the present disclosure may comprise creating, for each of a plurality of poses, including the first pose P₁ and the second pose P₂, a constellation model C(ϕ₀, ..., ϕₙ) representative of an arrangement of the first anatomical structure and an arrangement of the second anatomical structure as a function of the articulation parameters representative of the respective pose, particularly, as a function of a flexion angle ϕᵢ of the joint, so as to obtain a plurality of constellation models. The constellation model as such may be used as input data for creating and/or updating the statistical articulated joint model. The arrangement may be determined with respect to any suitable common reference.

According to the present disclosure, creating and/or updating the statistical articulated joint model may be based on the plurality of constellation models, in particular, may comprise combining the plurality constellation models. For example, the constellation model may be used for sorting medical images in image space for creating the joint model. Moreover, the constellation model may be used together with interpolation so as to obtain an articulated model that comprises poses for which no image data is available.

According to the present disclosure, the image registration may comprise applying a registration algorithm registering image data, e.g., based on landmarks in the image data, and/or meshes obtained by a segmentation algorithm, e.g., based on one or more vertices of a mesh. In particular, the method of the present disclosure may comprise, prior to the image registration, applying a segmentation algorithm segmenting the first anatomical structure and/or the second anatomical structure to obtain meshes. Any known segmentation and/or image registration methods may be applied. Anatomical atlas data may be employed.

The method of the present disclosure may comprise using the first articulation parameters and the second articulation parameters for a pre-registration.

For example, the first and second articulation parameters may be used for performing transformations that roughly match the anatomical structures prior to carrying out an image registration algorithm. This may reduce resources required for the image registration and may also resolve ambiguities that the image registration cannot resolve.

According to the present disclosure, the first anatomical structure may be used as a reference anatomical structure and the statistical articulated joint model may be representative of an arrangement of the second anatomical structure relative to the first anatomical structure.

For example, flexion angles may be expressed relative to the reference anatomical structure. The reference anatomical structure may be one of one or more bones of the joint.

According to the present disclosure, the statistical articulated joint model may be a model representative of one or more selected movement types and the method may comprise filtering data for one or more selected poses, in particular for one or more selected articulation parameters, representative of the one or more selected movement types, such that only data representative of the one or more selected movement types is used for creating and/or updating the statistical articulated joint model.

An articulated joint model that is representative of the selected movement type(s) may be advantageous for a detailed characterization of a joint. It may also reduce unnecessary complexity where only one movement type is relevant for a given application.

For example, a movement type may be a rotation and/or bending in only a first plane or bending only in a second plane. A movement can be represented by a collection of poses that are part of the movement. A movement type may, for example, be represented by only a subset of all possible poses of a joint. For example, a movement type may be represented only by poses having the same flexion angle in one direction and differing in the flexion angle in another direction. Accordingly, the articulation parameters representative of the selected movement type may be the articulation parameters corresponding to the selected poses representative of the selected movement type.

Filtering data for the selected poses may entail that only data corresponding to the selected poses, e.g., image data and/or meshes, are used as input for creating the articulated joint model.

The method of the present disclosure may comprise anatomical structure-wise registering of keypoints inferred from 3D surface image data of the body part to transform a reference segmentation of the first anatomical structure and/or of the second anatomical structure from the first pose P₁ to a third pose P₃ and creating and/or updating the statistical articulated joint model based on articulation parameters of the third pose P3 and the transformed reference segmentation and/or corresponding transformation data.

As an example, bones or other anatomical structures may have an impact on the surface of the subject, e.g., may be discernable through the skin and/or tissue. For example, protrusions may be caused on the surface. Points where an anatomical structure is discernable on the surface may be detected in the surface image data. The position of the detected points may then allow for estimating where one or more keypoints, e.g., on a mesh, of the anatomical structure are, particularly based on 3D image data and medical image data in a given pose optionally supplemented by general anatomical data. Based thereon, for other poses where no medical image data is available, detecting the points where the anatomical structure is discernable on the surface in 3D image data allows for inferring the positions of the keypoints. Based thereon, a transformation of the keypoints from one pose to the other pose can be determined and the mesh can be transformed as well based on the transformation of the keypoints. Thereby, additional data of the anatomical structure can be obtained even for poses where only surface image data is available. An example is described in more detail below.

The method of the present disclosure may comprise determining, in the first 3D surface image data of the body part, one or more keypoints of the surface of the body part in the first 3D surface image data corresponding to keypoints of the first anatomical structure and/or the second anatomical structure in the first medical image data.

A keypoint of the surface may be a point on the surface of a subject where part of the anatomical structure is discernable, e.g., by creating a protrusion on the surface. The corresponding keypoint of the anatomical structure may be a point of the surface of the part of the anatomical structure that is discernable on the surface, e.g., that causes a protrusion.

The method of the present disclosure may further comprise determining a position of each of the one or more keypoints of the surface of the body part and a position of each of the one or more keypoints of the first anatomical structure and/or second anatomical structure in the first pose P₁, for example by means of a/the reference segmentation of the first anatomical structure and/or a/the reference segmentation of the second anatomical structure.

The method of the present disclosure may further comprise processing third 3D surface image data of the body part arranged in a third pose P₃ to identify a position of each of the one or more keypoints of the surface of the body part in the third pose P₃. It is to be understood that the third pose may be different from second pose and different from the first pose.

The method of the present disclosure may further comprise, based on the position of each of the one or more keypoints of the surface of the body part in the third pose P₃, the position of each of the one or more keypoints of the surface of the body part in the first pose P₁, and the position of each of the one or more keypoints of the first anatomical structure and/or the second anatomical structure in the first pose P₁, determining a position of each of the corresponding keypoints of the first anatomical structure and/or the second anatomical structure in the third pose P₃.

The method of the present disclosure may further comprise determining keypoint transformation data representing one or more third transformations required to match each of the one or more keypoints of the first anatomical structure and/or the second anatomical structure in the first pose P₁ with each of the keypoints of the first anatomical structure and/or the second anatomical structure in the third pose P₃.

The method of the present disclosure may further comprise creating and/or updating the statistical articulated joint model based on the keypoint transformation data and third articulation parameters representative of the third pose P₃.

Thus, surface image data for poses for which no medical image data is available, e.g., the third pose, may be used as input for the articulated model. This allows for an even more extensive set of input data for the model, which also increases accuracy of the model.

The method of the present disclosure may comprise a step of checking for potential collisions of the reference segmentation of the first anatomical structure and the reference segmentation of the second anatomical structure when transforming from the first pose P₁ to the third pose P₃ and restricting movement of the anatomical structures accordingly.

This allows for avoiding extrapolation errors and/or other modelling errors. Specifically, where real anatomical structures do not collide, parts of the articulated model that represent a collision would be inaccurate and can therefore be discarded.

The method of the present disclosure may comprise simultaneously acquiring the first 3D surface image data and the first medical image data. That is, the method may comprise the steps of data acquisition of data used for creating and/or updating the model. This may be done by respective 3D surface imaging means and medical imaging means, e.g., as described above.

The invention also provides a data processing system to carry out and/or control any of the method steps of the present disclosure. In particular, the data processing system may be configured to carry out one or more, in particular all, of the computing steps of the present disclosure and/or to control acquiring the 3D surface image data and/or the medical image data, for example, by controlling a 3D surface imaging system configured to acquire the 3D surface image data and/or an imaging system configured to acquire the medical image data.

In particular, the data processing system may comprise one or more processors, in particular one or more computing devices and/or a distributed network of computing devices comprising the one or more processors, and the one or more processors may be configured to carry out and/or control the steps of the method of the present disclosure.

The invention also provides a system comprising the data processing system of the present disclosure and further comprising a 3D surface imaging system configured to acquire the 3D surface image data, including the first 3D surface image data and/or the second 3D surface image data and/or the third 3D surface image data. The system also comprises an imaging system configured to acquire medical image data, including the first medical image data and/or the second medical image data, in particular a CT imaging system and/or an MRT imaging system and/or an X-ray imaging system. The system may be configured to perform any of the methods of the present disclosure.

The invention also provides a computer program product comprising instructions which, when the program is executed by a computer, cause the computer to carry out and/or control any of the method steps described in the present disclosure.

The invention also provides a computer readable medium comprising instructions which, when executed by a computer, cause the computer to carry out and/or control any of the method steps described in the present disclosure.

The present disclosure also provides a use of the statistical articulated joint model for at least one of quality assurance for measurement data, properly placing/arranging a subject, properly measuring a subject, simulating and/or visualizing medical imaging arrangements, examining subject-specific articulation, deviations, and limitations.

The features and advantages outlined in the context of the method for modelling a joint similarly apply to the data processing system, the system comprising the data processing system, the computer program product, and the computer readable medium of the present disclosure.

Further features, examples, and advantages will become apparent from the detailed description making reference to the accompanying drawings.

### BRIEF DESCRIPTION OF THE DRAWINGS

Fig. 1 shows a schematic illustration of a system according to the present disclosure;
Figs. 2a and 2b shows schematic, not-to-scale representations of two different types of joints;
Fig. 3 shows a flow diagram representative of a method according to the present disclosure;
Figs. 4a and 4b show different views of different types of joints;
Figs 5a to 5c show examples for a 3D articulated model and a segmented view of a knee; and
Fig. 6 shows another exemplary flow chart for a method according to the present disclosure.

### DETAILED DESCRIPTION OF EMBODIMENTS

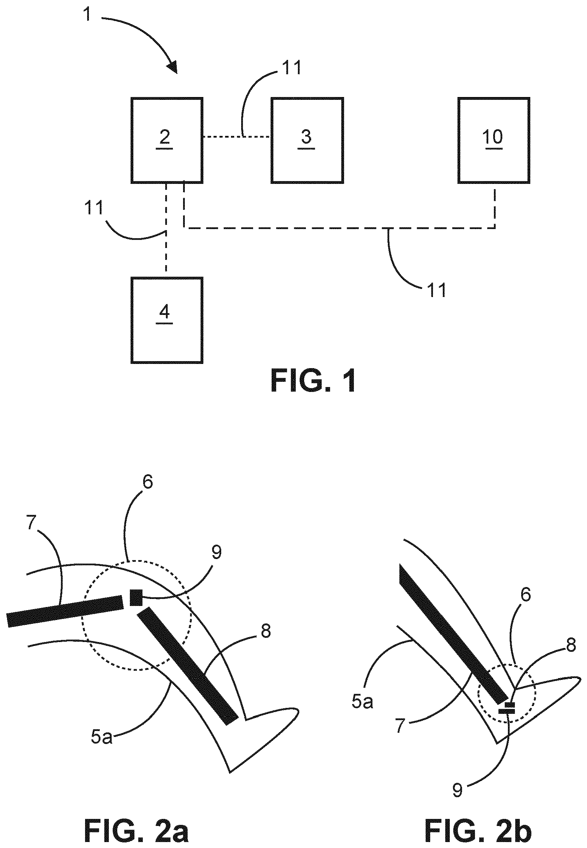

Fig. 1 illustrates a schematic representation of a system according to the present disclosure.

The system 1 comprises a data processing system 2 according to the present disclosure. The system may also comprise, as shown in the present example, a 3D surface imaging system 3, for example a depth camera, and an imaging system 4 configured to acquire 2D and/or 3D medical image data, for example, an MRI or CT scanner or an X-ray imaging device. The data processing system 2 may comprise one or more processors, in particular one or more computing devices and/or a distributed network of computing devices comprising the one or more processors.

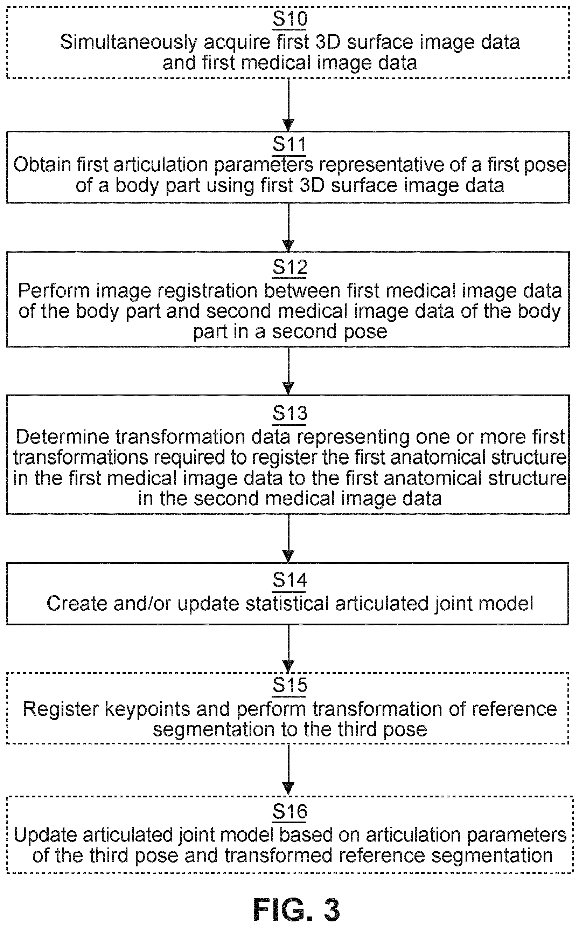

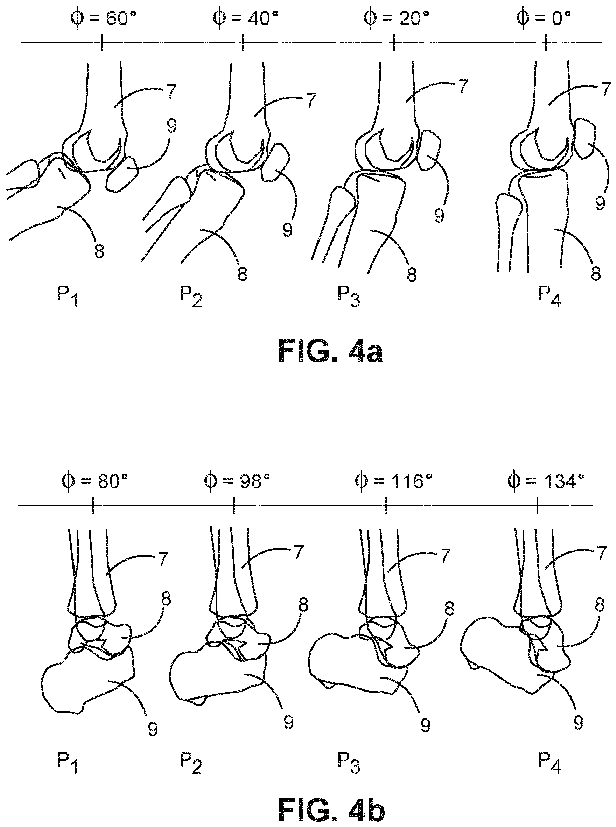

The system 1 is configured to carry out the method of the present disclosure, for example the method as specified in the method claims or as described below in the context of Figs. 3 and 4. In particular, the data processing system may be configured to carry out and/or control the method of the present disclosure, for example the method as specified in the method claims or as described below.

Specifically, the data processing system may be configured to process first 3D surface image data of a body part in a first pose by means of an automatic pose retrieval method so as to obtain first articulation parameters representative of the first pose. Exemplary body parts are shown in Figs. 2a and 2b and described in the context thereof, e.g., a leg and an ankle respectively. The body part comprises a first anatomical structure and a second anatomical structure connected by a joint, for example the bones and joints described in the context of Figs. 2a and 2b. The system may further be configured to perform image registration between first medical image data of the body part and second medical image data of the body part in a second pose. The first medical image data, e.g., CT, MRI, or X-ray image data, is image data acquired simultaneously with the first 3D surface image data and depicts the first anatomical structure and the second anatomical structure. The system is configured to perform the image registration individually for each of the first anatomical structure and the second anatomical structure. The system may further configured to determine transformation data representing one or more first transformations required to register the first anatomical structure in the first medical image data to the first anatomical structure in the second medical image data and/or one or more second transformations required to register the second anatomical structure in the first medical image data to the second anatomical structure in the second medical image data and to create and/or updating a statistical articulated joint model based at least on the transformation data, the first articulation parameters, and second articulation parameters representative of the second pose. If an articulated joint model is updated, the system may be configured to access a current statistical articulated joint model stored, e.g. locally or remotely, and update the accessed model. The system may then store the created and/or updated model, e.g., locally or remotely.

The 3D surface imaging system 3 may be configured to acquire the 3D surface image data used in the method claims or as described below. The medical imaging system may be configured to acquire medical image data, for example, CT, MRI, or X-ray image data as used in the method claims or as described below.

The data processing system, in addition to being configured to carry out the steps described above, may be configured to control image acquisition by means of the 3D surface imaging system and/or the medical imaging systems so as to obtain the respective image data. Alternatively or in addition, the processing system may be configured to retrieve previously stored image data and/or models, e.g., stored locally and/or remotely at one or more data storage devices 10. The data processing system may connect to the imaging system(s) and/or data storage system(s) where the image data and/or models are stored via data connections 11, e.g., wireless and/or wired data connections.

Figs. 2a and 2b are schematic, not-to scale representations of two different types of joints.

In Fig. 2a, as an example, the body part 5 is the lower limb of a human subject and the knee is shown as an example for the joint 6. The first anatomical structure 7 may be a femur and the second anatomical structure 8 may be a tibia. Moreover, the patella is shown as a third anatomical structure 9.

In Fig. 2b, as an example, the body part is the ankle of a human subject and the ankle joint is shown as an example for the joint 6. The tibia, talus bone, and calcaneus are shown as examples for the first, second and third anatomical structures 7 to 9. It is noted that the knee has only one degree of freedom and, accordingly, there is only one flexion angle for each pose. The ankle joint has two independent degrees of freedom and, accordingly, there are two flexion angles for each pose.

As described above in the general part of the description, a constellation model may be provided by the system and method of the present disclosure, which is a collection of the flexion angles for each of a plurality of poses. The constellation model may then be used to obtain the articulated joint model.

In Fig. 3, a flowchart illustrates an exemplary method for modelling a joint according to the present disclosure.

In step S11, first 3D surface image data of a body part in a first pose is processed by means of an automatic pose retrieval method so as to obtain first articulation parameters representative of the first pose. The body part comprises a first anatomical structure and a second anatomical structure connected by a joint.

In step S12, image registration between first medical image data of the body part and second medical image data of the body part in a second pose is performed. The first medical image data is image data acquired simultaneously with the first 3D surface image data and depicting the first anatomical structure and the second anatomical structure. The image registration is performed individually for each of the first anatomical structure and the second anatomical structure.

In the present example, the medical image data may be 3D medical image data, for example 3D CT or MRI image data. Accordingly, a 2D/3D registering of medical image data onto a mesh for obtaining 3D information may not be required in this case.

In step S13, transformation data representing one or more first transformations required to register the first anatomical structure in the first medical image data to the first anatomical structure in the second medical image data and/or one or more second transformations required to register the second anatomical structure in the first medical image data to the second anatomical structure in the second medical image data is determined.

In step S14, a statistical articulated joint model is created and/or updated based at least on the transformation data, the first articulation parameters, and second articulation parameters representative of the second pose.

In Fig. 3, optional steps S15 and S16 are shown. In step S15, an anatomical structure-wise, e.g., bone-wise, registration of keypoints of anatomical structures in the medical image data that may be inferred from 3D surface image data of the body part may be performed. For example, the shape of the surface of the body part may be indicative of the shape and position of a bone or other anatomical structure underneath the surface, such that detecting such shapes in the 3D surface image can be employed to estimate the position of keypoints of the respective anatomical structure in the medical image data. A reference segmentation of the first anatomical structure and/or of the second anatomical structure from the first pose to a third pose is transformed employing the estimated position of the keypoints.

In step S16, the articulated joint model is updated based on articulation parameters of the third pose and the transformed reference segmentation and/or corresponding transformation data. It is noted that steps S15 and S 16 may be performed after steps S11 to S14 or afterwards. If they are performed prior to steps S11 to S14, they may be used for creating a joint model, which may then be updated in step S14.

The optional steps S15 and S16 allow for incorporating information gained from 3D surface image data into the joint model even if no corresponding medical image data is available. This may be particularly useful when 3D surface image data within one imaging session are obtained at a higher rate than the medical image data.

It is noted that the method may optionally comprise the step S10 of simultaneously acquiring the 3D surface image data and the medical image data. However, alternatively, the image data may also at least in part be retrieved image data.

In the following, an exemplary method for modelling a joint according to the present disclosure will be described, wherein the medical images comprise 2D medical images. Most of the steps may, for example, be the same as in Fig. 3 and described above.

However, an additional step S11a is performed, wherein a 2D/3D registering of the 2D first and second medical image data is performed prior to the registration step S12. For example, the 2D medical image data may be registered onto a common mesh representative of a 3D shape of the surface of the respective anatomical structure, as will be described in more detail below.

The registration S 12 and the transformation step S13 may be carried out making use of the 2D/3D registered medical image data.

As an example, the above method may be applied where medical imaging modalities that depict anatomical structures, like bones, in a 2D projection are used, for example regular X-ray imaging systems. As mentioned above, 2D medical image data can be employed in the method according to the present disclosure for creating and/or updating the articulated joint model by performing a 2D/3D registering of the first and second 2D medical image data onto a common mesh topology. Thereby, the 2D image data can be accurately registered and incorporated into an articulated joint model. The 2D image data, in particular, can also be employed to create and/or update a 3D articulated joint model by performing the 2D/3D registration.

The mesh may be obtained from a pre-existing 3D model of each of the anatomical structures, for example a pre-existing 3D articulated model of the joint and/or a pre-existing rigid model of the joint or of the individual anatomical structures.

It is preferable to use the same mesh when repeating the 2D/3D registration for additional image data to be incorporated into the joint model. As an example, since the joint model is a statistical model that may incorporate data from several different subjects, a mean mesh for these subjects may be employed, which may be updated with each new additional medical image data set. An initial mesh may be created manually or semi-automatically or automatically.

The use of meshes for 2D/3D registration of anatomical structures can be performed using known method known in the art.

Figs. 4a and 4b illustrate different joints, in case of Fig. 4a the knee and in case of Fig. 4b the ankle, in different poses P1 to P4, respectively.

In Figs. 4a and 4b, the flexion angle ϕ is indicated for each pose. The knee has only one degree of freedom and, accordingly, there is only one flexion angle for each pose. The ankle joint has two independent degrees of freedom and, accordingly, there are two flexion angles for each pose. However only flexion of the upper ankle joint, i.e., dorsi- and plantarflexion, is shown for simplicity, i.e., the flexion angle for the lower ankle joint, i.e. the inversion or eversion angle, remains constant.

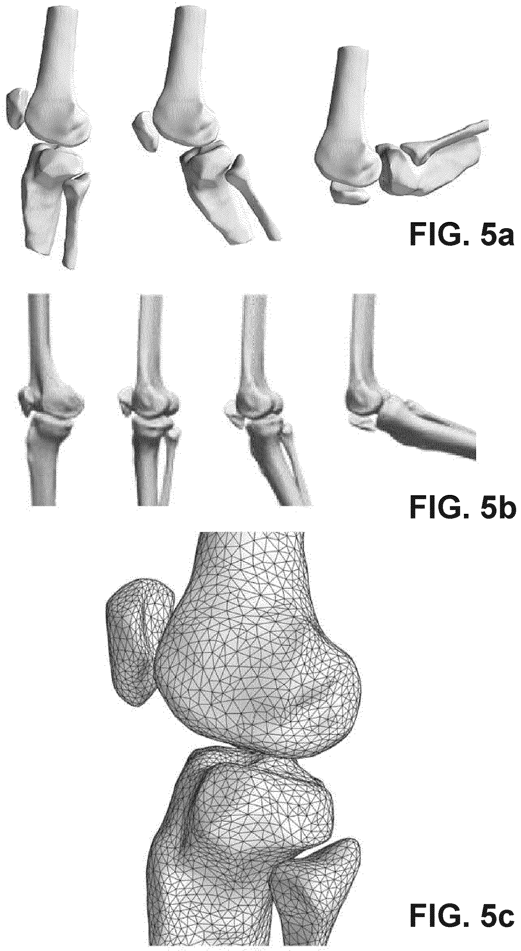

Figs. 5a and 5b show examples for a 3D articulated model of a knee. Fig. 5c shows a mesh, i.e., a segmented view of a knee, that may be used for different purposes for creating and/or updating the joint model, as described above.

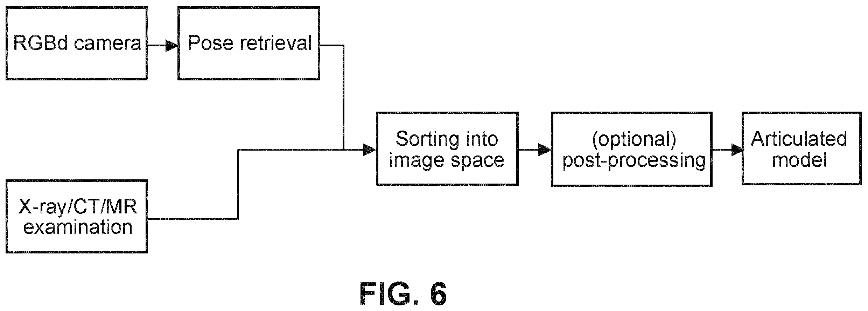

Fig. 6 shows another exemplary flow chart for a method according to the present disclosure. A medical imaging modality and a depth camera are used to simultaneously record medical image and depth image of the subject. This information is processed in a dedicated processing pipeline as shown in Fig. 6 in order to correctly co-register the medical images and generate or refine an articulated model.

The building blocks of an exemplary method are:
A 3D camera or range camera recording the subject during image formation.

A pose retrieval algorithm, e.g. known from computer games, for the joint of interest, especially deriving the flexion angle(s) *ϕ*_{0..*n*}.

A segmentation algorithm for segmenting the bone(s) within the image.

A registration algorithm registering images or meshes according to a prior (reference) bone using the measured poses of the respective bone.

A registration algorithm registering all other bones using the measured poses of the respective bones.

A translation/rotation transformation describing the relative movement of each bone.

Output: A constellation model C(*ϕ*₀, ..., *ϕₙ*) describing the position of model components for a given pose.

The above describes a fully automatic joint model construction from (depth-camera signal, medical image) pairs.

The registration in steps 4 and 5 may be based on the segmented bones, too, or both on the segmented bones and the estimated pose. In both cases, the depth-signal based pose estimation can be used for measuring the articulation parameters and thus for sorting the cases. Alternatively, the articulation parameters can be derived from the rotation degrees of freedom of the registration transformation (encoding rotations with respect to a reference pose).

The depth-based pose estimation can be used to filter a dataset for specific articulations, e.g. for selecting cases in which only the flexion-angle of the ankle joint is varied while the inversion/eversion angle is binned to a small, fixed range.

Instead of segmenting the bones in every image of the training cohort, the bone segmentation of some reference case can also be used for constructing the articulated bone model. In this case, these reference segmentations (e.g. in form of meshes) can be transformed to other poses by bone-wise registering the keypoints inferred from the depth signal. When transforming this pose to a different pose, an algorithm could check for potential collisions of the bone meshes and may then restrict the movement of the bones accordingly.

While the invention has been illustrated and described in detail in the drawings and foregoing description, such illustration and description are to be considered exemplary and not restrictive. The invention is not limited to the disclosed embodiments. In view of the foregoing description and drawings it will be evident to a person skilled in the art that various modifications may be made within the scope of the invention, as defined by the claims.

### LIST OF REFERENCE SIGNS:

System 1
Data processing system 2
3D surface imaging system 3
Imaging system 4
Body part 5
Joint 6
First anatomical structure 7
Second anatomical structure 8f
Third anatomical structure 9
Data storage device 10
Data connections 11
First pose P₁
Second pose P₂
Third pose P₃
Fourth pose P₄
Flexion angles ϕ

## Claims

1. A computer-implemented method for modelling a joint (6), the method comprising
processing (S11) first 3D surface image data of a body part (5) in a first pose (P₁) by means of an automatic pose retrieval method so as to obtain first articulation parameters representative of the first pose (P₁), wherein the body part (5) comprises a first anatomical structure (7) and a second anatomical structure (8) connected by a joint (6);
performing (S12) image registration between first medical image data of the body part (5), the first medical image data acquired simultaneously with the first 3D surface image data and depicting the first anatomical structure (7) and the second anatomical structure (8), and second medical image data of the body part (5) in a second pose (P₂), wherein the image registration is performed individually for each of the first anatomical structure (7) and the second anatomical structure (8);
determining (S13) transformation data representing one or more first transformations required to register the first anatomical structure (7) in the first medical image data to the first anatomical structure (7) in the second medical image data and/or one or more second transformations required to register the second anatomical structure (8) in the first medical image data to the second anatomical structure (8) in the second medical image data; and
creating and/or updating (S14) a statistical articulated joint model based at least on the transformation data, the first articulation parameters, and second articulation parameters representative of the second pose (P₂) derived from second 3D surface image data of the body part (5) obtained using automatic pose retrieval.

2. The method of claim 1,
wherein the statistical articulated joint model is based on transformation data, first articulation parameters, and second articulation parameters from image data acquired in multiple imaging sessions of a subject and/or from one or more imaging sessions of each of a plurality of subjects, and/or
wherein creating and/or updating the statistical articulated joint model comprises determining a mean over a randomly selected subset of the population and/or determining a mean over a subset of the population sharing a type of misalignment of the joint (6).

3. The method of claim 1 or 2, wherein the statistical articulated joint model is a 3D model.

4. The method of any of the preceding claims,
comprising creating, for each of a plurality of poses, including the first pose (P₁) and the second pose (P₂), a constellation model C(ϕ₀, ..., ϕₙ) representative of an arrangement of the first anatomical structure (7) and an arrangement of the second anatomical structure (8) as a function of the articulation parameters representative of the respective pose, particularly, as a function of a flexion angle (ϕᵢ) of the joint (6), so as to obtain a plurality of constellation models,
wherein creating and/or updating the statistical articulated joint model is based on the plurality of constellation models, in particular, comprises combining the plurality constellation models.

5. The method of any of the preceding claims,
wherein the image registration comprises applying a registration algorithm registering image data and/or meshes obtained by a segmentation algorithm, in particular, the method comprising, prior to the image registration, applying a segmentation algorithm segmenting the first anatomical structure (7) and/or the second anatomical structure (8) to obtain meshes, and/or
wherein the method comprises using the first articulation parameters and the second articulation parameters for a pre-registration.

6. The method of any of the preceding claims, wherein the first anatomical structure (7) is used as a reference anatomical structure and the statistical articulated joint model is representative of an arrangement of the second anatomical structure (8) relative to the first anatomical structure (7).

7. The method of any of the preceding claims, wherein the statistical articulated joint model is a model representative of one or more selected movement types and the method comprises filtering data for one or more selected poses, in particular for one or more selected articulation parameters, representative of the one or more selected movement types, such that only data representative of the one or more selected movement types is used for creating and/or updating the statistical articulated joint model.

8. The method of any of the preceding claims, further comprising
anatomical structure-wise registering (S15) of keypoints inferred from 3D surface image data of the body part (5) to transform a reference segmentation of the first anatomical structure (7) and/or of the second anatomical structure (8) from the first pose (P₁) to a third pose (P₃); and
creating and/or updating (S16) the statistical articulated joint model based on articulation parameters of the third pose (P₃) and the transformed reference segmentation and/or corresponding transformation data.

9. The method of any of the preceding claims, comprising
determining, in the first 3D surface image data of the body part (5), one or more keypoints of the surface of the body part (5) in the first 3D surface image data corresponding to keypoints of the first anatomical structure (7) and/or the second anatomical structure (8) in the first medical image data;
determining a position of each of the one or more keypoints of the surface of the body part (5) and a position of each of the one or more keypoints of the first anatomical structure (7) and/or second anatomical structure (8) in the first pose (P₁), for example by means of a/the reference segmentation of the first anatomical structure (7) and/or a/the reference segmentation of the second anatomical structure (8);
processing third 3D surface image data of the body part (5) arranged in a third pose (P₃) to identify a position of each of the one or more keypoints of the surface of the body part (5) in the third pose (P₃);
based on the position of each of the one or more keypoints of the surface of the body part (5) in the third pose (P₃), the position of each of the one or more keypoints of the surface of the body part (5) in the first pose (P₁), and the position of each of the one or more keypoints of the first anatomical structure (7) and/or the second anatomical structure (8) in the first pose (P₁), determining a position of each of the corresponding keypoints of the first anatomical structure (7) and/or the second anatomical structure (8) in the third pose (P₃);
determining keypoint transformation data representing one or more third transformations required to match each of the one or more keypoints of the first anatomical structure (7) and/or the second anatomical structure (8) in the first pose (P₁) with each of the keypoints of the first anatomical structure (7) and/or the second anatomical structure (8) in the third pose (P₃); and
creating and/or updating (S15) the statistical articulated joint model based on the keypoint transformation data and third articulation parameters representative of the third pose (P₃).

10. The method of claim 8 or claim 9, comprising a step of checking for potential collisions of the reference segmentation of the first anatomical structure (7) and the reference segmentation of the second anatomical structure (8) when transforming from the first pose (P₁) to the third pose (P₃) and restricting movement of the anatomical structures accordingly.

11. The method of any of the preceding claims comprising simultaneously acquiring (S10) the first 3D surface image data and the first medical image data.

12. A data processing system (2) configured to carry out the method steps of any one of claims 1 to 11.

13. A system (1) comprising the data processing system (2) of claim 12 and further comprising:
a 3D surface imaging system (3) configured to acquire 3D surface image data, including the first 3D surface image data and/or the second 3D surface image data and/or the third 3D surface image data; and
an imaging system (4) configured to acquire medical image data, including the first medical image data and/or the second medical image data, in particular a CT imaging system and/or an MRT imaging system and/or an X-ray imaging system.

14. A computer program product comprising instructions which, when the program is executed by a computer, cause the computer to carry out the method of any one of claims 1 to 11.

15. A computer readable medium comprising instructions which, when executed by a computer, cause the computer to carry out the method of any one of claims 1 to 11.

## Patentansprüche

1. Computerimplementiertes Verfahren zur Modellierung eines Gelenks (6), wobei das Verfahren umfasst:
Verarbeiten (S11) erster 3D-Oberflächenbilddaten eines Körperteils (5) in einer ersten Pose (P₁) mittels eines automatischen Posenabrufverfahrens, um erste Gelenkparameter zu erhalten, die repräsentativ für die erste Pose (P₁) sind, wobei der Körperteil (5) eine erste anatomische Struktur (7) und eine zweite anatomische Struktur (8) umfasst, die durch ein Gelenk (6) verbunden sind;
Durchführen (S12) einer Bildregistrierung zwischen ersten medizinischen Bilddaten des Körperteils (5), wobei die ersten medizinischen Bilddaten gleichzeitig mit den ersten 3D-Oberflächenbilddaten erfasst werden und die erste anatomische Struktur (7) und die zweite anatomische Struktur (8) darstellen, und zweiten medizinischen Bilddaten des Körperteils (5) in einer zweiten Pose (P₂), wobei die Bildregistrierung für jede der ersten anatomischen Struktur (7) und der zweiten anatomischen Struktur (8) einzeln durchgeführt wird;
Bestimmen (S13) von Transformationsdaten, die eine oder mehrere erste Transformationen darstellen, die erforderlich sind, um die erste anatomische Struktur (7) in den ersten medizinischen Bilddaten mit der ersten anatomischen Struktur (7) in den zweiten medizinischen Bilddaten zu registrieren, und/oder eine oder mehrere zweite Transformationen, die erforderlich sind, um die zweite anatomische Struktur (8) in den ersten medizinischen Bilddaten mit der zweiten anatomischen Struktur (8) in den zweiten medizinischen Bilddaten zu registrieren; und
Erstellen und/oder Aktualisieren (S14) eines statistischen Gelenkmodells, das zumindest auf den Transformationsdaten, den ersten Gelenkparametern und zweiten Gelenkparametern basiert, die repräsentativ für die zweite Pose (P₂) sind und aus zweiten 3D-Oberflächenbilddaten des Körperteils (5) abgeleitet sind, die mittels automatischem Posenabruf erhalten wurden.

2. Verfahren nach Anspruch 1,
wobei das statistische Gelenkmodell auf Transformationsdaten, ersten Gelenkparametern und zweiten Gelenkparametern aus Bilddaten basiert, die in mehreren Bildgebungssitzungen eines Subjekts und/oder aus einer oder mehreren Bildgebungssitzungen von jedem einer Vielzahl von Subjekten erfasst werden, und/oder
wobei Erstellen und/oder Aktualisieren des statistischen Gelenkmodells Bestimmen eines Mittelwerts über eine zufällig ausgewählte Teilmenge der Population und/oder Bestimmen eines Mittelwerts über eine Teilmenge der Population umfasst, die eine Art von Fehlstellung des Gelenks (6) gemein hat.

3. Verfahren nach Anspruch 1 oder 2, wobei es sich bei dem statistischen Gelenkmodell um ein 3D-Modell handelt.

4. Verfahren nach einem der vorstehenden Ansprüche,
umfassend, für jede einer Vielzahl von Posen, einschließlich der ersten Pose (P₁) und der zweiten Pose (P₂), Erstellen eines Konstellationsmodells C(ϕ₀, ..., ϕₙ), das repräsentativ für eine Anordnung der ersten anatomischen Struktur (7) und eine Anordnung der zweiten anatomischen Struktur (8) als eine Funktion der Gelenkparameter ist, die repräsentativ für die jeweilige Pose sind, insbesondere als eine Funktion eines Beugewinkels (ϕᵢ) des Gelenks (6), um eine Vielzahl von Konstellationsmodellen zu erhalten,
wobei Erstellen und/oder Aktualisieren des statistischen Gelenkmodells auf der Vielzahl von Konstellationsmodellen basiert und insbesondere Kombinieren der Vielzahl von Konstellationsmodellen umfasst.

5. Verfahren nach einem der vorstehenden Ansprüche,
wobei die Bildregistrierung Anwenden eines Registrierungsalgorithmus zum Registrieren von Bilddaten und/oder Netzen umfasst, die durch einen Segmentierungsalgorithmus erhalten werden, wobei das Verfahren insbesondere, vor der Bildregistrierung, Anwenden eines Segmentierungsalgorithmus zum Segmentieren der ersten anatomischen Struktur (7) und/oder der zweiten anatomischen Struktur (8) zum Erhalten von Netzen umfasst, und/oder
wobei das Verfahren Verwenden der ersten Gelenkparameter und der zweiten Gelenkparameter für eine Vorregistrierung umfasst.

6. Verfahren nach einem der vorstehenden Ansprüche, wobei die erste anatomische Struktur (7) als eine anatomische Referenzstruktur verwendet wird und das statistische Gelenkmodell repräsentativ für eine Anordnung der zweiten anatomischen Struktur (8) relativ zu der ersten anatomischen Struktur (7) ist.

7. Verfahren nach einem der vorstehenden Ansprüche, wobei das statistische Gelenkmodell ein Modell ist, das repräsentativ für einen oder mehrere ausgewählte Bewegungstypen ist, und das Verfahren Filtern von Daten für eine oder mehrere ausgewählte Posen umfasst, insbesondere für einen oder mehrere ausgewählte Gelenkparameter, die repräsentativ für den einen oder die mehreren ausgewählten Bewegungstypen sind, sodass nur Daten, die repräsentativ für den einen oder die mehreren ausgewählten Bewegungstypen sind, zum Erstellen und/oder Aktualisieren des statistischen Gelenkmodells verwendet werden.

8. Verfahren nach einem der vorstehenden Ansprüche, weiter umfassend:
anatomisches strukturbezogenes Registrieren (S15) von Schlüsselpunkten, die aus 3D-Oberflächenbilddaten des Körperteils (5) abgeleitet werden, um eine Referenzsegmentierung der ersten anatomischen Struktur (7) und/oder der zweiten anatomischen Struktur (8) von der ersten Pose (P₁) zu einer dritten Pose (P₃) zu transformieren; und
Erstellen und/oder Aktualisieren (S16) des statistischen Gelenkmodells basierend auf Gelenkparametern der dritten Pose (P₃) und der transformierten Referenzsegmentierung und/oder entsprechenden Transformationsdaten.

9. Verfahren nach einem der vorstehenden Ansprüche, umfassend:
Bestimmen, in den ersten 3D-Oberflächenbilddaten des Körperteils (5), eines oder mehrerer Schlüsselpunkte der Oberfläche des Körperteils (5) in den ersten 3D-Oberflächenbilddaten, die Schlüsselpunkten der ersten anatomischen Struktur (7) und/oder der zweiten anatomischen Struktur (8) in den ersten medizinischen Bilddaten entsprechen;
Bestimmen einer Position von jedem des einen oder der mehreren Schlüsselpunkte der Oberfläche des Körperteils (5) und einer Position von jedem des einen oder der mehreren Schlüsselpunkte der ersten anatomischen Struktur (7) und/oder der zweiten anatomischen Struktur (8) in der ersten Pose (P₁), zum Beispiel mittels einer/der Referenzsegmentierung der ersten anatomischen Struktur (7) und/oder einer/der Referenzsegmentierung der zweiten anatomischen Struktur (8);
Verarbeiten von dritten 3D-Oberflächenbilddaten des Körperteils (5), der in einer dritten Pose (P₃) angeordnet ist, um eine Position von jedem des einen oder der mehreren Schlüsselpunkte der Oberfläche des Körperteils (5) in der dritten Pose (P₃) zu identifizieren;
basierend auf der Position von jedem des einen oder der mehreren Schlüsselpunkte der Oberfläche des Körperteils (5) in der dritten Pose (P₃), der Position von jedem des einen oder der mehreren Schlüsselpunkte der Oberfläche des Körperteils (5) in der ersten Pose (P₁) und der Position von jedem des einen oder der mehreren Schlüsselpunkte der ersten anatomischen Struktur (7) und/oder der zweiten anatomischen Struktur (8) in der ersten Pose (P₁), Bestimmen einer Position von jedem der entsprechenden Schlüsselpunkte der ersten anatomischen Struktur (7) und/oder der zweiten anatomischen Struktur (8) in der dritten Pose (P₃);
Bestimmen von Schlüsselpunkt-Transformationsdaten, die repräsentativ für eine oder mehrere dritte Transformationen sind, die erforderlich sind, um jeden des einen oder der mehreren Schlüsselpunkte der ersten anatomischen Struktur (7) und/oder der zweiten anatomischen Struktur (8) in der ersten Pose (P₁) mit jedem der Schlüsselpunkte der ersten anatomischen Struktur (7) und/oder der zweiten anatomischen Struktur (8) in der dritten Pose (P₃) abzugleichen; und
Erstellen und/oder Aktualisieren (S15) des statistischen Gelenkmodells auf Basis der Schlüsselpunkt-Transformationsdaten und dritter Gelenkparametern, die repräsentativ für die dritte Pose (P₃) sind.

10. Verfahren nach Anspruch 8 oder Anspruch 9, umfassend einen Schritt des Überprüfens auf mögliche Kollisionen der Referenzsegmentierung der ersten anatomischen Struktur (7) und der Referenzsegmentierung der zweiten anatomischen Struktur (8) beim Transformieren von der ersten Pose (P₁) zu der dritten Pose (P₃), und entsprechendes Einschränken der Bewegung der anatomischen Strukturen.

11. Verfahren nach einem der vorstehenden Ansprüche, umfassend gleichzeitiges Erfassen (S10) der ersten 3D-Oberflächenbilddaten und der ersten medizinischen Bilddaten.

12. Datenverarbeitungssystem (2), konfiguriert zum Durchführen der Verfahrensschritte nach einem der Ansprüche 1 bis 11.

13. System (1), umfassend das Datenverarbeitungssystem (2) nach Anspruch 12, und weiter umfassend:
ein 3D-Oberflächenbildgebungssystem (3), das so konfiguriert ist, dass es 3D-Oberflächenbilddaten erfasst, einschließlich der ersten 3D-Oberflächenbilddaten und/oder der zweiten 3D-Oberflächenbilddaten und/oder der dritten 3D-Oberflächenbilddaten; und
ein Bildgebungssystem (4), das so konfiguriert ist, dass es medizinische Bilddaten erfasst, einschließlich der ersten medizinischen Bilddaten und/oder der zweiten medizinischen Bilddaten, insbesondere ein CT-Bildgebungssystem und/oder ein MRT-Bildgebungssystem und/oder ein Röntgenbildgebungssystem.

14. Computerprogrammprodukt, das Anweisungen umfasst, die bewirken, wenn das Programm von einem Computer ausgeführt wird, dass der Computer das Verfahren nach einem der Ansprüche 1 bis 11 durchführt.

15. Computerlesbares Medium, das Anweisungen umfasst, die bewirken, wenn sie von einem Computer ausgeführt werden, dass der Computer das Verfahren nach einem der Ansprüche 1 bis 11 durchführt.

## Revendications

1. Procédé mis en œuvre par ordinateur de modélisation d'une articulation (6), le procédé comprenant
le traitement (S11) de premières données d'image de surface 3D d'une partie du corps (5) dans une première pose (P₁) au moyen d'un procédé de récupération de pose automatique de manière à obtenir de premiers paramètres d'articulation représentatifs de la première pose (P₁), dans lequel la partie du corps (5) comprend une première structure anatomique (7) et une deuxième structure anatomique (8) connectées par une articulation (6) ;
la réalisation (S12) d'un enregistrement d'image entre de premières données d'image médicale de la partie du corps (5), les premières données d'image médicale acquises simultanément avec les premières données d'image de surface 3D et la représentation de la première structure anatomique (7) et la deuxième structure anatomique (8), et de deuxièmes données d'image médicale de la partie du corps (5) dans une deuxième pose (P₂), dans lequel l'enregistrement d'image est réalisé individuellement pour chacune de la première structure anatomique (7) et de la deuxième structure anatomique (8) ;
la détermination de données de transformation (S13) représentant une ou plusieurs premières transformations nécessaires pour enregistrer la première structure anatomique (7) dans les premières données d'image médicale sur la première structure anatomique (7) dans les deuxièmes données d'image médicale et/ou une ou plusieurs deuxièmes transformations nécessaires pour enregistrer la deuxième structure anatomique (8) dans les premières données d'image médicale sur la deuxième structure anatomique (8) dans les deuxièmes données d'image médicale ; et
la création et/ou mise à jour (S14) d'un modèle d'articulation statistique basé au moins sur les données de transformation, les premiers paramètres d'articulation et de deuxièmes paramètres d'articulation représentatifs de la deuxième pose (P₂) dérivés de deuxièmes données d'image de surface 3D de la partie du corps (5) obtenues en utilisant une récupération de pose automatique.

2. Procédé selon la revendication 1,
dans lequel le modèle d'articulation statistique est basé sur des données de transformation, de premiers paramètres d'articulation et de deuxièmes paramètres d'articulation à partir de données d'image acquises dans de multiples séances d'imagerie d'un sujet et/ou à partir d'une ou de plusieurs séances d'imagerie pour chacun d'une pluralité de sujets, et/ou
dans lequel la création et/ou mise à jour du modèle d'articulation statistique comprend la détermination d'une moyenne sur un sous-ensemble sélectionné aléatoire de la population et/ou la détermination d'une moyenne sur un sous-ensemble de la population partageant un type de désalignement de l'articulation (6).

3. Procédé selon la revendication 1 ou 2, dans lequel le modèle d'articulation statistique est un modèle 3D.

4. Procédé selon l'une quelconque des revendications précédentes,
comprenant la création, pour chacune d'une pluralité de poses, incluant la première pose (P₁) et la deuxième pose (P₂), d'un modèle de constellation C(ϕ₀, ..., ϕₙ) représentatif d'un agencement de la première structure anatomique (7) et d'un agencement de la deuxième structure anatomique (8) en fonction des paramètres d'articulation représentatifs de la pose respective, en particulier en fonction d'un angle de flexion (ϕᵢ) de l'articulation (6), de manière à obtenir une pluralité de modèles de constellation,
dans lequel la création et/ou mise à jour du modèle d'articulation statistique est basée sur la pluralité des modèles de constellation et en particulier comprend la combinaison de la pluralité de modèles de constellation.

5. Procédé selon l'une quelconque des revendications précédentes,
dans lequel l'enregistrement d'image comprend l'application d'un algorithme d'enregistrement enregistrant des données d'image et/ou maillages obtenus par un algorithme de segmentation, en particulier le procédé comprenant, avant l'enregistrement d'image, l'application d'un algorithme de segmentation segmentant la première structure anatomique (7) et/ou la deuxième structure anatomique (8) pour obtenir des maillages, et/ou
dans lequel le procédé comprend l'utilisation des premiers paramètres d'articulation et des deuxièmes paramètres d'articulation pour un pré-enregistrement.

6. Procédé selon l'une quelconque des revendications précédentes, dans lequel la première structure anatomique (7) est utilisée comme structure anatomique de référence et le modèle d'articulation statistique est représentatif d'un agencement de la deuxième structure anatomique (8) par rapport à la première structure anatomique (7).

7. Procédé selon l'une quelconque des revendications précédentes, dans lequel le modèle d'articulation statistique est un modèle représentatif d'un ou de plusieurs types de mouvement sélectionnés et le procédé comprend le filtrage de données pour une ou plusieurs poses sélectionnées, en particulier pour un ou plusieurs paramètres d'articulation sélectionnés, représentatifs de l'un ou des plusieurs types de mouvement sélectionnés, de sorte que seules des données représentatives de l'un ou des plusieurs types de mouvement sélectionnés sont utilisées pour la création et/ou mise à jour du modèle d'articulation statistique.

8. Procédé selon l'une quelconque des revendications précédentes, comprenant en outre
l'enregistrement structurel anatomique (S15) de points clés déduits à partir de données d'image de surface 3D de la partie du corps (5) pour transformer une segmentation de référence de la première structure anatomique (7) et/ou de la deuxième structure anatomique (8) de la première pose (P₁) à une troisième pose (P₃) ; et
la création et/ou mise à jour (S16) du modèle d'articulation statistique sur la base de paramètres d'articulation de la troisième pose (P₃) et la segmentation de référence transformée et/ou des données de transformation correspondantes.

9. Procédé selon l'une quelconque des revendications précédentes, comprenant
la détermination, dans les premières données d'image de surface 3D de la partie du corps (5), d'un ou de plusieurs points clés de la surface de la partie du corps (5) dans les premières données d'image de surface 3D correspondant à des points clés de la première structure anatomique (7) et/ou la deuxième structure anatomique (8) dans les premières données d'image médicale ;
la détermination d'une position de chacun de l'un ou des plusieurs points clés de la surface de la partie du corps (5) et une position de chacun de l'un ou des plusieurs points clés de la première structure anatomique (7) et/ou deuxième structure anatomique (8) dans la première pose (P₁), par exemple au moyen d'une/de la segmentation de référence de la première structure anatomique (7) et/ou d'une/de la segmentation de référence de la deuxième structure anatomique (8) ;
le traitement de données d'image de surface 3D de la partie du corps (5) agencée dans une troisième pose (P₃) pour identifier une position de chacun de l'un ou des plusieurs points clés de la surface de la partie du corps (5) dans la troisième pose (P₃) ;
sur la base de la position de chacun de l'un ou des plusieurs points clés de la surface de la partie du corps (5) dans la troisième pose (P₃), la position de chacun de l'un ou des plusieurs points clés de la surface de la partie du corps (5) dans la première pose (P₁), et la position de chacun de l'un ou des plusieurs points clés de la première structure anatomique (7) et/ou la deuxième structure anatomique (8) dans la première pose (P₁), la détermination d'une position de chacun des points clés correspondants de la première structure anatomique (7) et/ou de la deuxième structure anatomique (8) dans la troisième pose (P₃) ;
la détermination de données de transformation de point clé représentant une ou plusieurs troisièmes transformations nécessaires pour mettre en correspondance chacun de l'un ou des plusieurs points clés de la première structure anatomique (7) et/ou de la deuxième structure anatomique (8) dans la première pose (P₁) avec chacun des points clés de la première structure anatomique (7) et/ou de la deuxième structure anatomique (8) dans la troisième pose (P₃) ; et
la création et/ou mise à jour (S15) du modèle d'articulation statistique sur la base des données de transformation de point clé et de troisièmes paramètres d'articulation représentatifs de la troisième pose (P₃).

10. Procédé selon la revendication 8 ou la revendication 9, comprenant une étape de vérification de collisions potentielles entre la segmentation de référence de la première structure anatomique (7) et la segmentation de référence de la deuxième structure anatomique (8) lors de la transformation de la première pose (P₁) à la troisième pose (P₃) et de restriction de mouvement des structures anatomiques en conséquence.

11. Procédé selon l'une quelconque des revendications précédentes comprenant l'acquisition simultanée (S10) des premières données d'image de surface 3D et des premières données d'image médicale.

12. Système de traitement de données (2) configuré pour réaliser les étapes de procédé selon l'une quelconque des revendications 1 à 11.

13. Système (1) comprenant le système de traitement de données (2) selon la revendication 12 et comprenant en outre :
un système d'imagerie de surface 3D (3) configuré pour acquérir des données d'image de surface 3D, incluant les premières données d'image de surface 3D et/ou les deuxièmes données d'image de surface 3D et/ou les troisièmes données d'image de surface 3D ; et
un système d'imagerie (4) configuré pour acquérir des données d'image médicale, incluant les premières données d'image médicale et/ou les deuxièmes données d'imagerie médicale, en particulier un système d'imagerie CT et/ou un système d'imagerie MRT et/ou un système d'imagerie à rayons X.

14. Produit de programme informatique comprenant des instructions qui, lorsque le programme est exécuté par un ordinateur, amènent l'ordinateur à réaliser le procédé selon l'une quelconque des revendications 1 à 11.

15. Support lisible par ordinateur comprenant des instructions qui, lorsqu'elles sont exécutées par un ordinateur, amènent l'ordinateur à réaliser le procédé selon l'une quelconque des revendications 1 à 11.
